(19) Europäisches Patentamt
European Patent Office
Office européen des brevets

(11) Publication number: **0 228 113 A2**

(12) **EUROPEAN PATENT APPLICATION**

(21) Application number: 86202127.6

(22) Date of filing: 01.12.86

(51) Int. Cl.[4]: **A 61 K 31/495**, A 61 K 31/535, A 61 K 31/44, A 61 K 31/445, A 61 K 31/505, A 61 K 31/415, A 61 K 31/41

(30) Priority: 03.12.85 NL 8503341

(43) Date of publication of application: 08.07.87 Bulletin 87/28

(84) Designated Contracting States: AT BE CH DE ES FR GB GR IT LI LU NL SE

(71) Applicant: Nederlandse Organisatie voor Toegepast Natuurwetenschappelijk Onderzoek TNO, J. van Stolberglaan 148, NL-2595 CL Den Haag (NL)

(72) Inventor: Kerkenaar, Antonius, Plaggewagen 12, NL-1261 KG Blaricum (NL)

(74) Representative: van der Beek, George Frans et al, Nederlandsch Octrooibureau Johan de Wittlaan 15 P.O. Box 29720, NL-2502 LS 's-Gravenhage (NL)

(54) **Use of sterol-biosynthesis inhibitors in combating certain gram-negative bacteria.**

(57) The use of sterol-biosynthesis-inhibitors as active substances in combating gram-negative bacteria having iso- and ante-iso-fatty acids in their membranes. An example of such gram-negative bacteria is for instance Legionella pneumophila being the cause of the feared Legionnaire's disease. Besides the pharmacological use the above meant inhibitors can also exercise their activity against said gram-negative bacteria in aqueous systems like cool water systems etc. . . .

EP 0 228 113 A2

Use of sterol-biosynthesis-inhibitors as active substances in combating microorganisms of a specific type as well as compositions suitable for combating such microorganisms.

TITLE MODIFIED see front page

The invention relates to the use of one or more sterol-biosynthesis-inhibitors as active substances in combating microorganisms of a specific type.

The use of sterol-biosysthesis-inhibitors in combating several fungi is known for a long time. A review of sterol-biosynthesis-inhibitors investigated in this respect is mentioned in "Fungicide Resistance in Crop Protection", Dekker, J. and Georgopoulos, S.G., Pudoc, Wageningen, 1980, pages 71-86 and 87-100. However, it cannot be deduced from said reference that such sterol-biosynthesis-inhibitors might have a possible activity against for instance a specific type of gram-negative bacteria.

Furthermore it is indicated in "Pesticide Biochemistry and Physiology" 12, 195-204 (1979) and "Pesticide Science" 15, 188-198 (1984) that a number of gram-positive bacteria in contrast to gram-negative bacteria are more or less sensitive to sterol-biosynthesis-inhibitors. On account of such results it was generally accepted that gram-negative bacteria are insensitive to such inhibitors.

Surprisingly it has been found that sterol-biosynthesis-inhibitors are active against gram-negative bacteria which have iso- and ante-iso--fatty acids in their membranes. More in particular the invention relates for instance to the use of sterol-biosynthesis-inhibitors as active substances for the manufacture of pharmaceutical compositions for combating (pathogenic) gram-negative bacteria having iso- and ante-iso--fatty acids in their membranes. Because of the fact that gram-negative bacteria can be combated much more difficultly than gram-positive bacteria the invention can be considered a breakthrough in the combat of many infections which were up to now hardly treatable and which were caused by gram-negative bacteria with iso- and anti-iso-fatty acids in their membranes. An example of such a bacterium which is not or hardly combatable is Legionella pneumophila being the cause of the feared Legionnaire's disease.

Except Legionella pneumophila also other examples of gram-negative bacteria with iso- and ante-iso-fatty acids in their membranes can be mentioned: unicellular 'gliding' bacteria from the class of Flexibacteriae like Capnocytophaga and Myxococcus spp.; Bacteroides spp.;

Desulfovibrio spp.; Flavobacterium meningosepticum and F. thalpophilum; Sphingobacterium spp.; Streptomyces spp.; Spirochaeta spp.; and Pseudomonas maltophila.

Based on the general concept of the invention it also relates to the use of sterol-biosynthesis-inhibitors for combating gram-negative bacteria with iso- and ante-iso-fatty acids in their membranes which are present in aqueous systems like cool water installations, air-conditioning systems as well as in the off-shore industry where especially the combat of sulphate-reducing bacteria like Desulfovibrio vulgaris, D. sulfuricans and other species have a high priority. Said sulphate-reducing bacteria convert sulphate in $H_2S$ which has both toxical and corrosive properties. In this respect it is pointed for instance at hollow piles of an oilrig filled with seawater which is internally attacked by corrosive materials like the above mentioned $H_2S$. The souring of oildeposits can also be due to the conversion of sulphate into $H_2S$.

The term sterol-biosynthesis-inhibitors indicated above comprises a heterogeneous group of chemical substances that despite their heterogeneousness have three characteristics in common: they all have at least one nitrogen-containing ring; with some exceptions they contain at least one asymmetric carbon atom; and they all interfere with sterol-biosynthesis by inhibiting steps after the formation of lanosterol like $C_{14}$-demethylation, $\Delta^{14}$-reduction, $\Delta^8 \longrightarrow \Delta^7$ isomerisation and so on.

With regard to the term "iso- and ante-iso-fatty acids" the following is reported. Terminally methyl-branched-chain fatty acids are grouped in three series based on their biosynthetic relationships (T.Kaneda, Bacteriol. Rev. 41, 391-418 (1977)). The definitions of iso- and ante-iso permit only iso-methyl substituted fatty acids (an iso--ethyl would be an ante-iso-methyl of one higher chain number) but would allow both methyl and ethyl as ante-iso-substitutions. Only the methyl ante-iso substituent has so far been found in nature, although bacteria can synthesize ethyl-ante-iso-compounds when appropriate precursors are provided. The above meant three series of branched-chain fatty acids can be illustrated as follows:

- Ante-iso series $CH_3-CH_2-CH-CH_2-(CH_2)_n-COOH$*
  $CH_3$
- Odd-numbered iso series $CH_3-CH-CH_2-CH_2-(CH_2)_n-COOH$*
  $CH_3$
- Even-numbered iso series $CH_3-CH-CH_2-(CH_2)_n-COOH$*
  $CH_3$

* n = 9 or 11

Examples of sterol-biosynthesis-inhibitors which can be used according to the invention are:

- triforine (formula 1)

1.

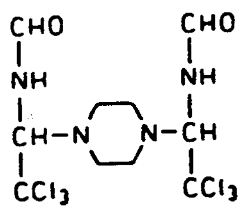

- dodemorph (formula 2)

2.

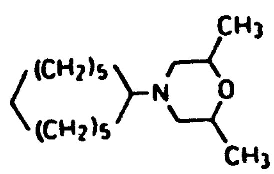

- fenpropimorph (formula 3),

3.

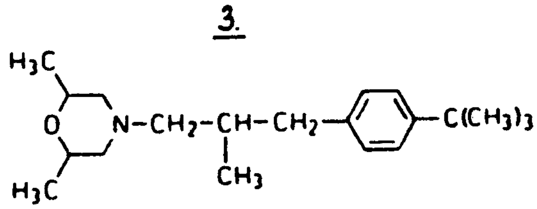

- tridemorph (formula 4)

4.

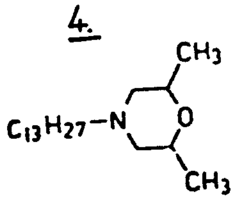

- Ro 4767/002 (formula 5)

5.

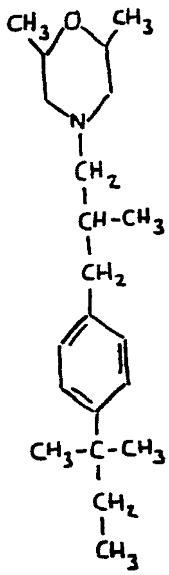

- buthiobate (formula 6)

6.

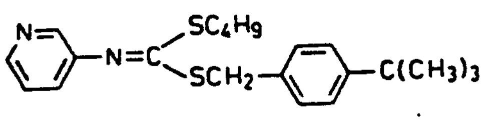

- EL-241 (formula 7)

7.

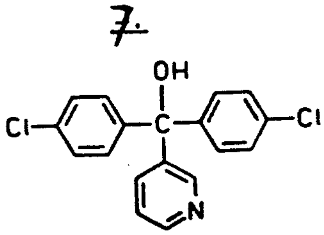

- fenpropidin (formula 8)

8.

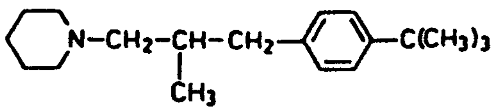

- ancymidol (formula 9)

9.

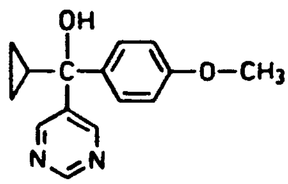

- fenarimol (formula 10)

10.

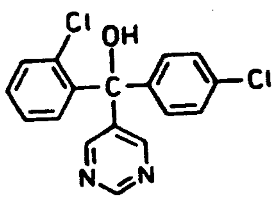

- nuarimol (formula 11)

11.

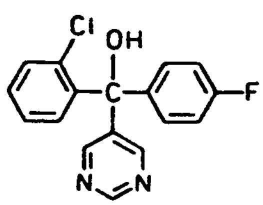

- triarimol (formula 12)

12.

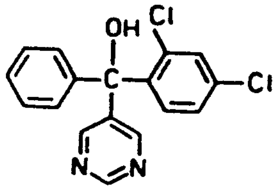

- clotrimazole (formula 13)

13.

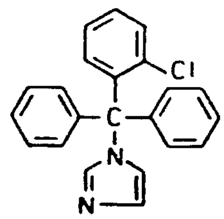

- N-dodecylimidazole (formula 14)

14.

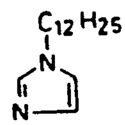

- econazole (formula 15)

15.

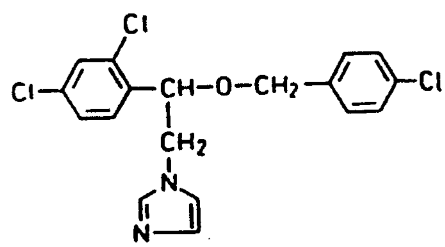

- ketoconazole (formula 16)

16.

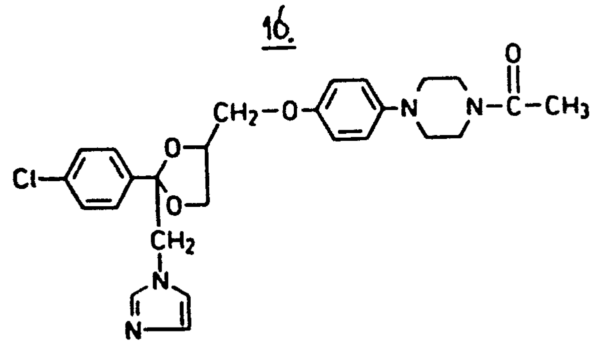

- XE-326 (formula 17)

17.

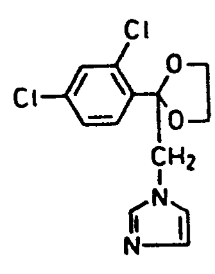

- miconazole (formula 18)

18.

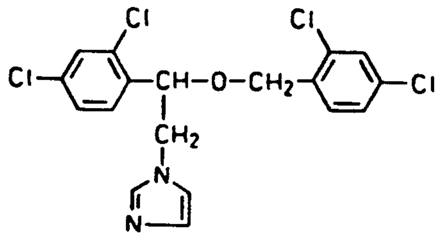

- phenapronil (formula 19)

19.

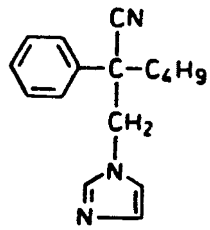

- prochloraz (formula 20)

20.

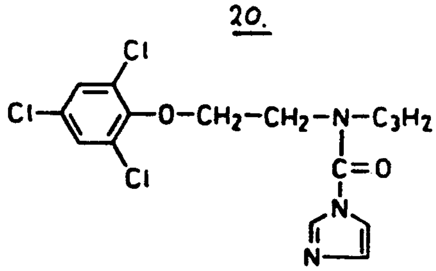

- triflumazole (formula 21)

21.

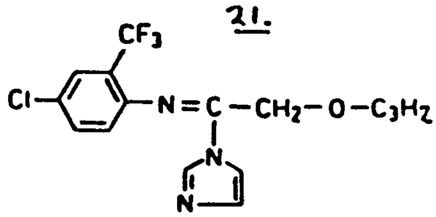

- imazalil (formula 22)

22.

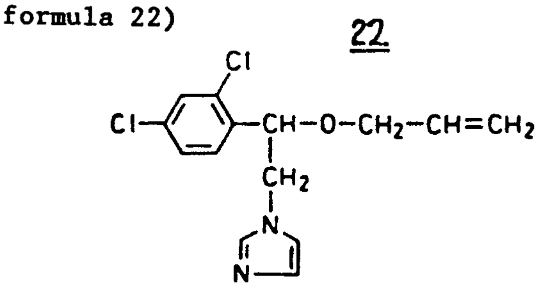

- lombazole (formula 23)

23.

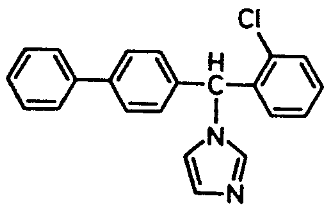

- bifonazole (formula 24)

24.

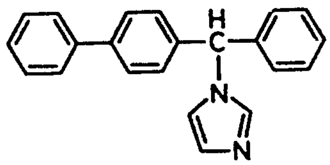

- BAY 1-9139 (formula 25)

25.

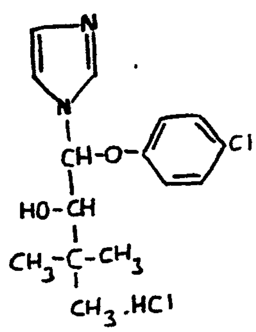

- isoconazole (formula 26)

26.

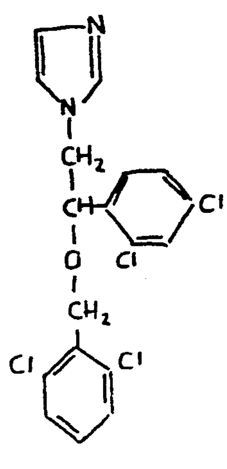

- tioconazole (formula 27)

27.

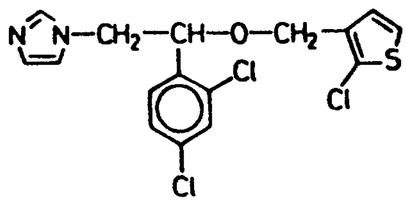

- sulconazole (formula 28)

28.

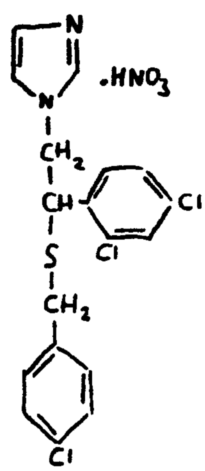

- oxiconazole (formula 29)

29.

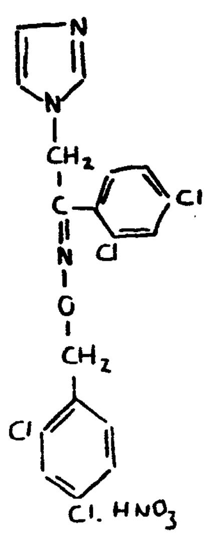

- butoconazole (formula 30)

30

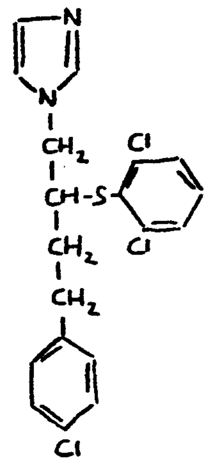

- bitertanol (formula 31)

31.

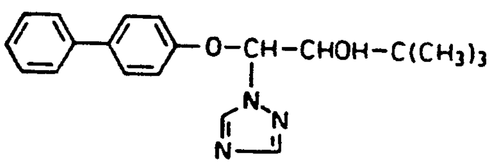

- CGA-64250 (propiconazole; formula 32)

32.

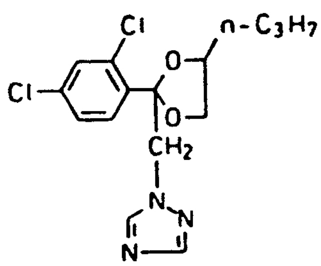

- CGA-64251 (etaconazole; formula 33)

33.

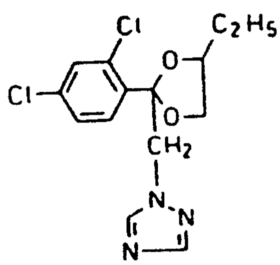

- fluotrimazole (formula 34)

34.

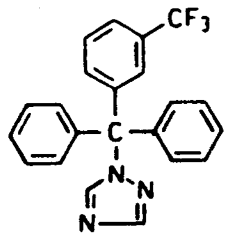

- triadimefon (formula 35)

35.

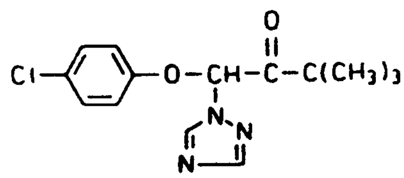

- triadimenol (formula 36)

36.

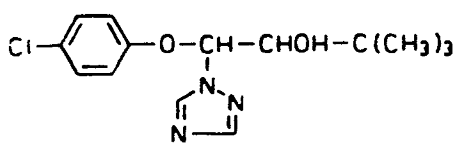

- dicyclobutrazole (formula 37)

37.

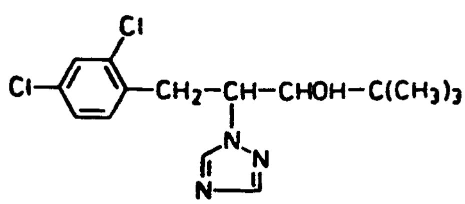

- diniconazole (formula 38)

38.

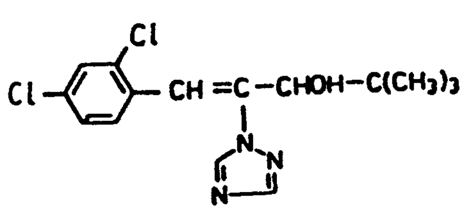

- itraconazole (formula 39)

39.

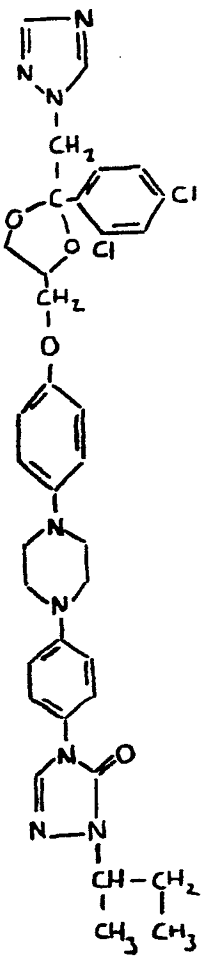

- terconazole (formula 40)

40.

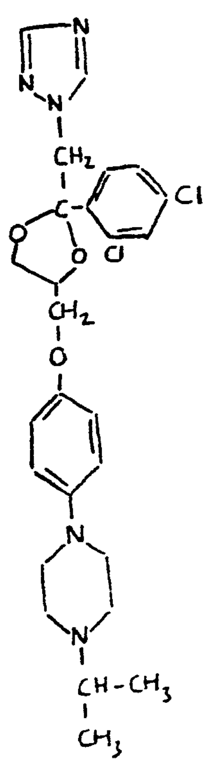

- ICI 153,066 (formula 41)

41.

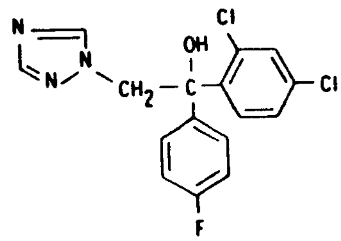

- vibunazole (formula 42)

42.

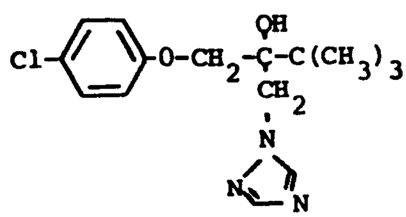

The bacteria with iso- and ante-iso-fatty acids in their membranes are preferably combated with sterol-biosynthesis-inhibitors in the form of azole compounds.

Hereunder three examples of in-vitro-tests are illustrated according to which the MIC-values* of the compounds usable according to the invention are determined.

( *MIC-value: 'minimal inhibitory concentration' i.e. lowest concentration whereby microscopically no visible growth can be detected).

Example I

The in-vitro-tests are carried out with 18 strains of Legionella spp..

The MIC-values are determined by measuring the growth of bacteria in Petri-dishes with 'buffered charcoal yeast-extract agar' nutrient. This nutrient has been described in an article of Feeley J.C. et al. in J.Clin.Microbiol. 10 (1979), 437-441. The dishes were inocculated with a suspension of bacteria which were cultivated in the same medium in culture-tubes during 1 week. The incubation was carried out at 37°C in the dark.

The inhibitors were added to the agar medium being just liquid by means of solutions in acetone graduated in concentration. The final concentration was at most 1%. Acetone was added to the control. Then the liquid was poured out in Petri-dishes and was inocculated in the above described way after solidification.

The concentration range of each compound was within 1 and 128 µg/ml with rising steps of 1, 2, 4, 8 etc..

De MIC-values were determined 48 hours after inocculation.

The results of the MIC-determinations are reported in the undermentioned Table A.

TABLE A

MIC-determinations in μg/ml on 'buffered charcoal yeast-extract agar' (pH 6,9; 48 hours; 37°C).

| strain | MIC (μg/ml) | | | |
|---|---|---|---|---|
| | lombazole | bifonazole | clotrimazole | miconazole |
| L.longbeachae gr. 1 | 64 | 64 | 16 | 128 |
| L.longbeachae gr. 2 | 64 | 64 | 16 | 128 |
| L.dumoffii | 64 | 64 | 16 | 128 |
| L.jordanis | 64 | 64 | 16 | 128 |
| L.gormanii | 64 | 128 | 8 | 128 |
| L.bozemanii gr. 1 | 128 | 128 | 8 | 128 |
| L.wadsworthii | 32 | 32 | 8 | 128 |
| L.oakridgensis | 64 | 64 | 8 | 64 |
| L.micdadei | 64 | 64 | 16 | 128 |
| L.pneumophila gr. 2 | 128 | 128 | 16 | 128 |
| L.pneumophila gr. 3 | 128 | 128 | 8 | 128 |
| L.pneumophila gr. 4 | 128 | 128 | 16 | 128 |
| L.pneumophila gr. 5 | 64 | 64 | 16 | 128 |
| L.pneumophila gr. 6 | 64 | 64 | 16 | 128 |
| L.pneumophila gr. 7 | 128 | 128 | 16 | 128 |
| L.pneumophila gr. 8 | 128 | 128 | 16 | 128 |
| L.pneumophila gr. 9 | 128 | 128 | 16 | 128 |
| L.pneumophila gr.10 | 64 | 128 | 16 | 128 |

gr. 1, 2, 3 etc. are sero types.

Example II

The in-vitro-tests are carried out with 2 sero types of Legionella pneumophila. The conditions were the same as mentioned in Example I, with the exception of the concentration range of each substance and the incubation time. The concentration range of each compound was between 1 and 100 μg/ml with rising steps of 1, 2, 5, 10, etc.; the incubation time was four days.

The results of the MIC-determinations are reported in the undermentioned Table B.

0228113

TABLE B

MIC-determinations in µg/ml on 'buffered charcoal yeast-extract agar' (pH 6,9; 4 days; 37°C).

| substance | Legionella pneumophila | |
|---|---|---|
| | gr. 1 | gr. 5 |
| tridemorph | 100 | 50 |
| fenarimol | 100 | 100 |
| etaconazole | > 100 | > 100 |

gr. 1 and 5 are sero types.

Example III

The in-vitro-tests were carried out with Myxococcus xanthus and Pseudomonas maltophila. The MIC-values were determined in Petri-dishes with casitone 1%, $KH_2PO_4$ 1,4%, $MgSO_4$ 0,2%, agar 1,5% (pH 7,6), as nutrient for Myxococcus and a synthetic amino acid medium with 1,5% agar described in an article of Hoeprich et al. in Antimicrob.Agents Chemother. 1970 (1971), 494-497 as nutrient for Pseudomonas. The conditions were the same as described in Example II with the exception of the incubation times and temperature. The incubation time for Myxococcus was four days and for Pseudomonas two days; the incubation temperature was 30°C.

The results of the MIC-determinations for Myxococcus xanthus are reported in the undermentioned Table C. The MIC-value of lombazole for Pseudomonas maltophila was 5 µg/ml.

TABLE C

MIC-determinations in µg/ml

| substance | Myxococcus xanthus |
|---|---|
| lombazole | 2 |
| clotrimazole | 2 |
| miconazole | 2 |

# CLAIMS

1. The use of one or more sterol-biosynthesis-inhibitors as active substances in combating gram-negative bacteria having iso- and ante-iso--fatty acids in their membranes.

2. The use of one or more sterol-biosynthesis-inhibitors as active substances for the manufacture of pharmaceutical compositions in combating gram-negative bacteria having iso- and ante-iso-fatty acids in their membranes.

3. The use of one or more sterol-biosynthesis-inhibitors as active substances for the manufacture of pharmaceutical compositions in combating Legionella pneumophila.

4. The use of one or more sterol-biosynthesis-inhibitors with the formulae 1-42 as active substances for the manufacture of pharmaceutical compositions in combating gram-negative bacteria having iso- and ante--iso-fatty acids in their membranes.

5. The use of one or more sterol-biosynthesis-inhibitors as active substances for the manufacture of compositions in combating gram-negative bacteria having iso- and ante-iso-fatty acids in their membranes in aqueous systems.

6. The use of one or more sterol-biosynthesis-inhibitors having formulae 1-42 as active substances for the manufacture of compositions in combating gram-negative bacteria with iso- and ante-iso-fatty acids in their membranes in aqueous systems.

7. Pharmaceutical composition for combating gram-negative bacteria having iso- and ante-iso-fatty acids in their membranes, characterized in that the composition contains as active substance(s) one or more sterol-biosynthesis-inhibitors as well as a pharmacologically acceptable carrier.

8. Pharmaceutical composition according to claim 7, characterized in that the composition contains as active substance(s) one or more of the sterol-biosynthesis-inhibitors with the formulae 1-42.

9. Composition for combating gram-negative bacteria with iso- and ante-iso-fatty acids in their membranes in aqueous systems, characterized in that the composition contains as active substance(s) one or more sterol-biosynthesis-inhibitors as well as a carrier suitable for such an application.

10. Composition according to claim 9, characterized in that the composition contains as active substance(s) one or more sterol-biosynthesis-inhibitors with the formulae 1-42.

***